# EUROPEAN PATENT SPECIFICATION

(11) **EP 4 201 237 B1**
(45) Date of publication and mention of the grant of the patent: **25.03.2026**
(21) Application number: 22213862.0
(22) Date of filing: 15.12.2022
(51) Int. Cl.: A24F 40/46, H05B 3/04, H05B 3/42

(54) **HEATING ASSEMBLY AND ELECTRONIC ATOMIZER**
HEIZANORDNUNG UND ELEKTRONISCHER ZERSTÄUBER
ENSEMBLE DE CHAUFFAGE ET ATOMISEUR ÉLECTRONIQUE

(30) Priority: 27.12.2021 CN 202123320550 U
(43) Date of publication of application: 28.06.2023
(73) Proprietor: Shenzhen Smoore Technology Limited, Shenzhen, Guangdong 518102 (CN)
(72) Inventor: LI, Jun, Shenzhen, 518102 (CN); ZENG, Zhaohuan, Shenzhen, 518102 (CN)
(74) Representative: Manitz Finsterwald Patent- und Rechtsanwaltspartnerschaft mbB

(56) References cited:
- EP-A1- 3 280 280
- EP-B1- 3 280 280
- WO-A1-2020/201295
- WO-A1-2021/069526
- US-A1- 2021 100 289

## Description

### TECHNICAL FIELD

The present invention relates to the field of vaporization technologies, and in particular, to a heating assembly and an electronic atomizer.

### BACKGROUND

Aerosol is a colloidal dispersion system formed by solid or liquid small particles dispersed and suspended in an air medium. Since the aerosol can be absorbed by a human body through a respiratory system, a novel alternative absorption manner is provided for a user. For example, an electronic vaporization device that generates aerosols through an aerosol substrate can be used in different fields such as the medical field, to deliver inhalable aerosols to the user, thereby replacing a conventional product form and an absorption manner.

Currently, an electronic atomizer configured to bake a solid aerosol-generation substrate is usually provided with a heating cavity configured to accommodate the aerosol-generation substrate. When using the electronic atomizer, the user needs to insert the aerosol-generation substrate into the heating cavity, and then presses a start button to start the electronic atomizer to bake the aerosol-generation substrate, resulting in relatively cumbersome operations. Related technologies can be found in US 2021/100289 A1, EP 3 280 280 A1, WO 2021/069526 A1, and WO 2020/201295 A1.

### SUMMARY

Accordingly, it is necessary to provide a heating assembly and an electronic atomizer to resolve the problems that a detection method for detecting insertion of an aerosol-generation substrate is relatively complex and reliability of a detection structure is relatively poor. By using the heating assembly and the electronic atomizer, technical effects of simplifying the detection method and improving the reliability of the detection structure can be achieved. The present invention is set out in the appended claims.

According to an aspect of this application, a heating assembly is provided, which is configured to heat the aerosol-generation substrate. The heating assembly includes a heating cavity and the heating cavity includes: a first conductive region; a second conductive region, provided on a side of the first conductive region; and an insulating region, provided between the first conductive region and the second conductive region and configured to electrically isolate the first conductive region from the second conductive region; wherein in a state where the aerosol-generation substrate is accommodated in the heating cavity, an electrical connection is formed between the first conductive region and the second conductive region through the aerosol-generation substrate.

In an embodiment, the heating cavity includes a first end and a second end that are arranged opposite to each other. The aerosol-generation substrate is inserted into the second end from the first end, and the first conductive region is provided on a side of the second conductive region facing the second end.

In an embodiment, the first conductive region is provided at the second end of the heating cavity, and the second conductive region is provided on a cavity side wall of the heating cavity.

In an embodiment, one end of the second conductive region is connected to the insulating region, and the other end of the second conductive region extends to the first end of the heating cavity in a direction away from the insulating region.

In an embodiment, the heating assembly further includes a first structural member, a second structural member, and an insulating member, wherein the first structural member forms a part of a cavity bottom wall of the heating cavity, and at least a part of the first structural member forms the first conductive region; the second structural member forms a part of a cavity side wall of the heating cavity, and at least a part of the second structural member forms the second conductive region; and the insulating member is arranged between the first structural member and the second structural member, and the insulating member forms the insulating region.

In an embodiment, the first structural member is made of a conductive material, and/or the second structural member is made of a conductive material.

In an embodiment, the insulating member includes an insulating bottom wall and an insulating side wall formed by extending from the insulating bottom wall in the same direction. The insulating side wall surrounds the insulating bottom wall in a circumferential direction to form an insulating accommodating cavity. The insulating bottom wall is provided with a mounting hole in communication with the insulating accommodating cavity. The first structural member is accommodated in the mounting hole.

In an embodiment, an inner diameter of the mounting hole is less than an inner diameter of the insulating accommodating cavity.

According to an aspect of this application, an electronic atomizer is provided, including a battery assembly and the foregoing heating assembly. The heating assembly is electrically connected to the battery assembly.

In an embodiment, the electronic atomizer further includes a vibration unit. When the first structural member and the second structural member transmit electrical signals through the aerosol-generation substrate, the vibration unit vibrates.

By providing the first conductive region, the second conductive region, and the insulating region in the heating cavity, the heating assembly can accurately detect whether there is an aerosol-generation substrate accommodated in the heating cavity without a manual operation by a user, which is high in ease of use and reliability, and prevents the aerosol-generation substrate from being excessively squeezed, thereby eliminating the risk of damaging the aerosol-generation substrate. In addition, a structure of a heating cylinder is simple, and therefore there is no need to add too many components such as an electromagnetic induction coil, thereby enabling production cost to be lower and assembly efficiency to be higher.

### BRIEF DESCRIPTION OF THE DRAWINGS

FIG. 1 is a schematic diagram of a heating assembly according to an embodiment of the present invention;
FIG. 2 is a schematic diagram of the heating assembly shown in FIG. 1 to which an aerosol-generation substrate is inserted;
FIG. 3 is a schematic diagram of a heating assembly according to another embodiment of the present invention; and
FIG. 4 is a schematic diagram of a heating assembly according to still another embodiment of the present invention.

### DETAILED DESCRIPTION

To make the foregoing objectives, features and advantages of the present invention more comprehensible, detailed description is made to specific implementations of the present invention below with reference to the accompanying drawings. In the following description, many specific details are described to give a full understanding of the present invention. However, the present invention may be implemented in many other manners different from those described herein. A person skilled in the art may make similar improvements without departing from the connotation of the present invention as set out in the appended claims.

In the description of the present invention, it should be understood that orientation or position relationships indicated by the terms such as "length", "above", "below", "vertical", "horizontal", "top", "bottom", "inner", "outer", "clockwise", "counterclockwise", "axial", "radial", and "circumferential" are based on orientation or position relationships shown in the accompanying drawings, and are merely used for describing the present invention and simplifying the description, rather than indicating or implying that the mentioned apparatus or element should have a particular orientation or be constructed and operated in a particular orientation. Therefore, such terms should not be construed as limiting of the present invention.

In addition, the terms "first" and "second" are used for descriptive purposes only and are not to be construed as indicating or implying relative importance or implicitly indicating the number of technical features indicated. Therefore, features defining "first" and "second" can explicitly or implicitly include at least one of the features. In the description of the present invention, unless otherwise explicitly defined, "a plurality of" means at least two, for example, two, three, and the like.

In the present invention, unless otherwise explicitly specified and defined, terms such as "mounted", "connected", "connection", and "fixed" should be understood in broad sense, for example, the connection may be a fixed connection, a detachable connection, or an integral connection; or the connection may be a mechanical connection or an electrical connection; or the connection may be a direct connection, an indirect connection through an intermediary, or internal communication between two elements or a mutual action relationship between two elements, unless otherwise specified explicitly. A person of ordinary skill in the art can understand specific meanings of the terms in the present invention according to specific situations.

In the present invention, unless explicitly specified or limited otherwise, a first feature being located "above" or "below" a second feature may be the first feature being in a direct contact with the second feature, or the first feature being in an indirect contact with the second feature through an intermediate medium. Moreover, the first feature "over", "above" and "up" the second feature may be that the first feature is directly above or obliquely above the second feature, or simply indicates that a horizontal height of the first feature is higher than that of the second feature. The first feature "under", "below" and "down" the second feature may be that the first feature is directly below or obliquely below the second feature, or simply indicates that a horizontal height of the first feature is less than that of the second feature.

It should be noted that, when an element is referred to as "being fixed to" or "being arranged on" another element, the element may be directly on the other element, or an intermediate element may be present. When an element is considered to be "connected to" another element, the element may be directly connected to the other element, or an intermediate element may also be present. The terms "vertical", "horizontal", "above", "below", "left", "right" and similar expressions used in this specification are only for purposes of illustration but not indicate a unique implementation.

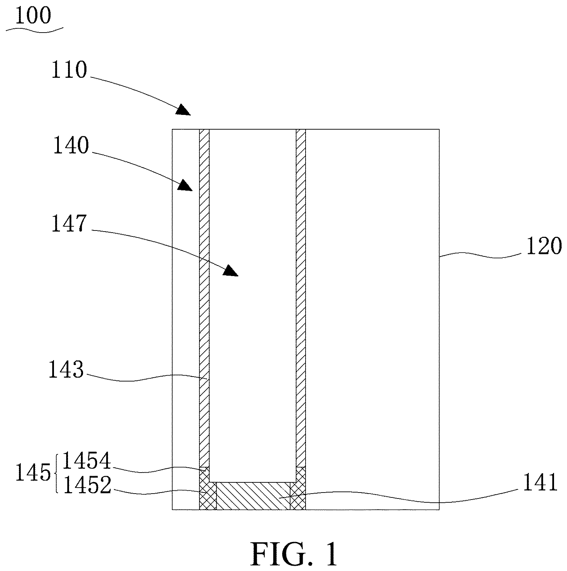

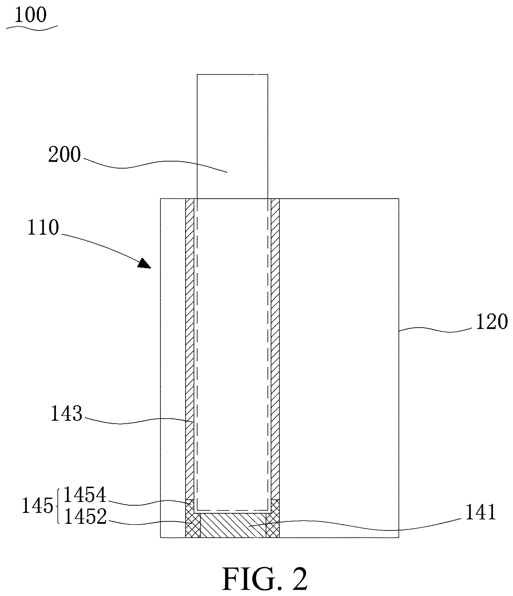

Referring to FIG. 1 and FIG. 2, an embodiment of the present invention provides an electronic atomizer 100, including a battery assembly (not shown) and a heating assembly 110. A solid aerosol-generation substrate 200 may be inserted into the heating assembly 110, and the heating assembly 110 can heat the aerosol-generation substrate 200 under an action of electrical energy of a battery, so as to generate aerosols for a user to inhale. Specifically, in the following embodiments, the aerosol-generation substrate 200 is in a cylindrical shape.

As described in the background, the method used by the conventional electronic atomizer for detecting whether an aerosol-generation substrate is inserted is relatively complex and has low reliability, which hinders the promotion and application of the electronic atomizer to some extent.

To resolve the above problems, still referring to FIG 1 and FIG. 2, the heating assembly 110 includes a main housing 120, which includes a heating cavity 147 with an opening at one end. The heating cavity 147 is configured to accommodate the aerosol-generation substrate 200. The electronic atomizer 100 further includes a detection unit (not shown). The detection unit is electrically connected to the heating assembly 110, and is configured to determine whether there is the aerosol-generation substrate 200 accommodated in a heating cylinder 140, thereby controlling an operating state of the electronic atomizer 100.

Specifically, the heating cavity 147 includes a first conductive region, a second conductive region, and an insulating region. The second conductive region is provided on a side of the first conductive region. The first conductive region and the second conductive region can respectively be used as a transceiver for performing capacitive sensing and a transceiver for performing resistance measurement, so as to receive and transmit a capacitive sensing signal and a resistance change signal. The insulating region is provided between the first conductive region and the second conductive region and is configured to electrically isolate the first conductive region from the second conductive region. The detection unit is configured to detect the capacitive sensing signal and the resistance signal between the first conductive region and the second conductive region, and to output an insertion signal according to the capacitive sensing signal and the resistance signal.

When there is no aerosol-generation substrate 200 accommodated in the heating cavity 147, the first conductive region is electrically isolated from the second conductive region by the insulating region. Therefore, the first conductive region and the second conductive region cannot form an electrical signal loop. In this case, the capacitive sensing signal and the resistance signal between the first conductive region and the second conductive region are obtained by the detection unit, and are respectively marked as C1 and R1. When there is the aerosol-generation substrate 200 accommodated in the heating cavity 147, the aerosol-generation substrate 200 improves electrical conductivity of the first conductive region and the second conductive region. An electrical connection is formed between the first conductive region and the second conductive region through the solid aerosol-generation substrate 200, thereby changing magnitude of the capacitive sensing signal and the resistance signal between the first conductive region and the second conductive region. In this case, the capacitive sensing signal and the resistance signal between the first conductive region and the second conductive region are obtained by the detection unit, and are respectively marked as C2 and R2. The detection unit obtains differences (that is, a difference between C1 and C2, and a difference between R1 and R2) between values measured before and after by filtering through an algorithm, then determines whether there is the aerosol-generation substrate 200 accommodated in the heating cavity 147 by determining sizes of the measurement differences and a preset calibration difference.

In this way, by providing the first conductive region, the second conductive region, and the insulating region in the heating cavity 147, the heating assembly 110 can accurately detect whether there is the aerosol-generation substrate 200 accommodated in the heating cavity 147 without a manual operation by a user, which is high in ease of use and reliability, and prevents the aerosol-generation substrate 200 from being excessively squeezed, thereby eliminating the risk of damaging the aerosol-generation substrate 200. In addition, a structure of the heating assembly 110 is simple, and therefore there is no need to add too many components such as an electromagnetic induction coil, thereby reducing the production cost and increasing the assembly efficiency.

In some embodiments, the detection unit is a capacitance detection chip or a resistance detection chip. The detection unit is usually in a standby state, and is woke up at regular intervals to perform detection. An interval time preferably ranges from 0.1 second to 1 second. In an optional embodiment, if the detection unit detects the existence of the aerosol-generation substrate 200, detection is performed repeatedly for many times. If the existence aerosol-generation substrate 200 is detected each time, it is determined that there is the aerosol-generation substrate 200 accommodated in the heating cavity 147, thus outputting the insertion signal to cause the heating assembly 110 to generate heat to heat the aerosol-generation substrate 200.

In some embodiments, the heating cavity 147 includes a first end and a second end that are arranged opposite to each other. The first end is an end of the heating cavity 147 having an opening end that is in communication with an external environment, and the second end is an end of the heating cavity 147 provided with a cavity bottom wall. The aerosol-generation substrate is inserted into the second end from the first end of the heating cavity 147. The first conductive region is provided on a side of the second conductive region facing the second end. That is, during insertion of the aerosol-generation substrate 200, the aerosol-generation substrate 200 first comes into contact with the first conductive region, and then it comes into contact with the second conductive region. Finally, a part of the aerosol-generation substrate 200 is in contact with the first conductive region, and the other part of the aerosol-generation substrate 200 is in contact with the second conductive region, so as to implement the electrical connection between the first conductive region and the second conductive region. Since the electrical connection between the first conductive region and the second conductive region can be implemented only when the aerosol-generation substrate 200 is simultaneously in contact with the first conductive region and the second conductive region, a range of a heating area of the aerosol-generation substrate 200 may be controlled by setting a position of the second conductive region, so as to implemented different heating requirements.

As a preferred implementation, the first conductive region is provided at the second end of the heating cavity 147, and the second conductive region is provided on a cavity side wall of the heating cavity 147. In addition, one end of the second conductive region is connected to the insulating region, and the other end of the second conductive region extends to the second end of the heating cavity 147 in a direction away from the insulating region. Since the first conductive region is provided at the second end of the heating cavity 147, when and only when the aerosol-generation substrate 200 is inserted into the bottom of the heating cavity 147 to be in contact with the first conductive region, the first conductive region can be electrically connected to the second conductive region through the solid aerosol-generation substrate 200. In this case, it is avoided that the aerosol-generation substrate 200 is heated when the aerosol-generation substrate 200 is not inserted in place, thereby preventing a position of a heating area from offsetting because the aerosol-generation substrate 200 is not completely inserted into the heating cavity 147. Therefore, the heating effect of the heating assembly 110 is ensured.

As shown in FIG. 1, specifically, in some embodiments, the heating assembly 110 includes the heating cylinder 140 arranged in the main housing 120. The heating cylinder 140 has a substantially cylindrical shape to form the heating cavity 147. Specifically, the heating cylinder 140 includes a first structural member 141, a second structural member 143, and an insulating member 145. The first structural member 141 forms a part of the cavity bottom wall of the heating cavity 147, and at least a part of the first structural member 141 forms the first conductive region. The second structural member 143 forms a part of the cavity side wall of the heating cavity 147, and at least a part of the second structural member 143 forms the second conductive region. The insulating member 145 is arranged between the first structural member 141 and the second structural member 143, and the insulating member 145 forms the insulating region.

Specifically, the first structural member 141 has a solid cylindrical shape and is entirely made of a conductive material. An upper surface of the first structural member 141 forms a part of the cavity bottom wall of the heating cavity 147, and the first structural member 141 forms the first conductive region.

The insulating member 145 has a hollow rotating body shape. The insulating member 145, which is entirely made of an insulating material, includes an insulating bottom wall 1452 and an insulating side wall 1454 formed by extending from the insulating bottom wall 1452 in the same direction. The insulating side wall 1454 surrounds the insulating bottom wall 1452 in a circumferential direction to form an insulating accommodating cavity. The insulating bottom wall 1452 is provided with a mounting hole in communication with the insulating accommodating cavity. The mounting hole and the insulating accommodating cavity are coaxially arranged, and an inner diameter of the mounting hole is less than an inner diameter of the insulating accommodating cavity. The first structural member 141 is accommodated in the mounting hole. An upper surface of the insulating bottom wall 1452 surrounds the upper surface of the first structural member 141 to form the other part of the cavity bottom wall. An inner surface of the insulating side wall 1454 forms a part of the cavity side wall of the heating cavity 147. The insulating member 145 forms the insulating region.

The second structural member 143 has a hollow tubular shape and is entirely made of a conductive material. One end of the second structural member 143 is connected to the insulating side wall 1454, and the other end of the second structural member 143 extends vertically in a direction away from the first structural member 141, so as to form a hollow cylindrical structure and be in communication with the insulating accommodating cavity. In addition, an inner diameter of the second structural member 143 is the same as an inner diameter of the insulating member 145. An inner surface of the second structural member 143 forms the other part of the cavity bottom wall of the heating cavity 147, and the second structural member 143 forms the second conductive region.

In this way, the insulating member 145, the first structural member 141, and the second structural member 143 jointly form the heating cavity 147 with an opening at one end. The insulating bottom wall 1452 and the first structural member 141 jointly form the cavity bottom wall of the heating cavity 147, and the insulating side wall 1454 and the second structural member 143 jointly from the cavity side wall of the heating cavity 147. One end of the aerosol-generation substrate 200 passes through the second structural member 143 and abuts against the first structural member 141. The second structural member 143 is wrapped on an outer circular surface of the aerosol-generation substrate 200, so that the aerosol-generation substrate 200 may serve to increase a resistance value and a capacitance value between the first structural member 141 and the second structural member 143. A size of the first structural member 141 and a size of the mounting hole matching the first structural member may be adjusted as required, so as to further adjust a minimum gap between the first structural member 141 and the second structural member 143, thereby avoiding a mistaken triggering caused by an excessive short distance between the first structural member 141 and the second structural member 143.

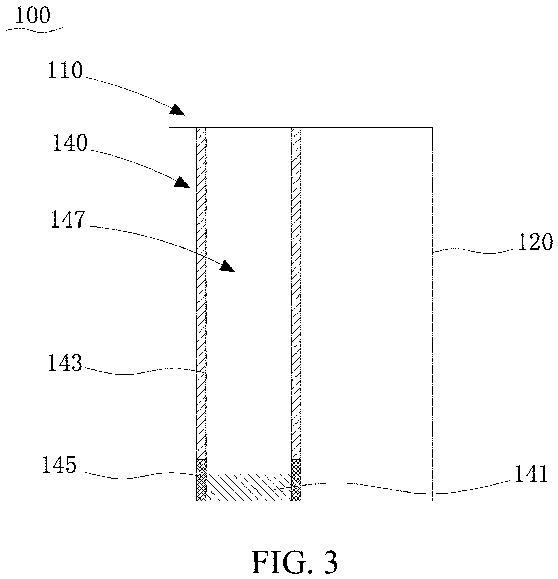

As shown in FIG. 3, in some other embodiments, the insulating member 145 has a tubular shape with openings at two ends, and inner diameters of the insulating member 145 are equal everywhere. The first structural member 141 is accommodated in the insulating member 145. In addition, an axial length of the insulating member 145 is greater than an axial length of the insulating member 145, and the insulating member 145 protrudes from an end of the first structural member 141 facing the opening end of the heating cavity 147.

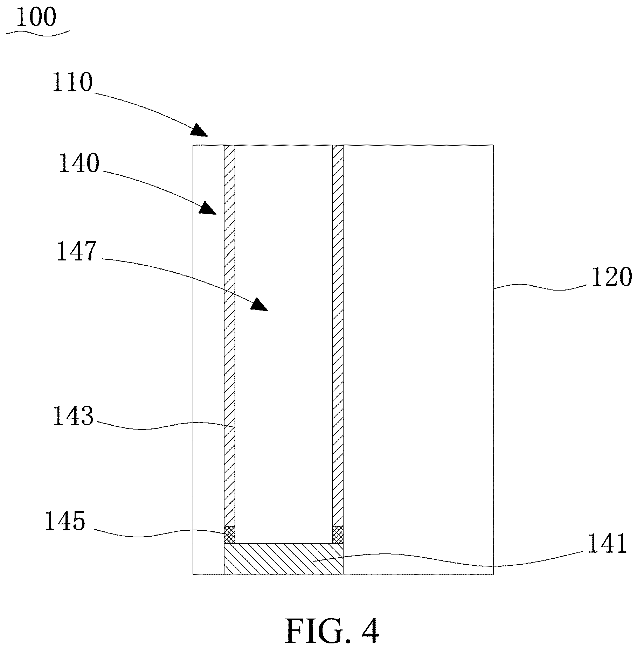

As shown in FIG. 4, in some other embodiments, the insulating member 145 is provided between the first structural member 141 and the second structural member 143. The insulating member 145 is in a tubular shape with the openings at two ends. The first structural member 141 and the second structural member 143 abut against the two opposite ends of the insulating member 145, respectively.

In some embodiments, both the first conductive region and the second conductive region are formed by a conductive film formed in the main housing 120, and in this case, the first structural member 141 and the second structural member 143 can be omitted.

In some embodiments, the electronic atomizer 100 further includes a vibration unit (not shown). When the first structural member 141 is electrically connected to the second structural member 143 through the solid aerosol-generation substrate 200, the vibration unit vibrates, so as to remind the user that the aerosol-generation substrate 200 has been inserted into a correct position, thereby preventing the user from continuing to exert a force to cause the aerosol-generation substrate 200 to be deformed and damaged after the aerosol-generation substrate 200 is in contact with the first structural member 141.

The heating assembly 110 and the electronic atomizer 100 performs insertion detection by using the principle that the aerosol-generation substrate 200 can increase the electrical conductivity between the first conductive region and the second conductive region. The heating assembly 110 and the electronic atomizer 100 have simple structures and relatively high reliability, thus causing no damage to the aerosol-generation substrate 200. In addition, the heating assembly 110 can accurately detects whether the aerosol-generation substrate 200 is inserted into the bottom portion of the heating cavity 147, and provides a feedback to the user through vibration, thereby preventing the heating effect from being affected because the heating position is offset, and preventing the aerosol-generation substrate 200 from being deformed due to an excessive force exerted by the user, and ensuring good inhaling taste.

## Claims

1. An electronic atomizer (100), comprising an aerosol-generation substrate (200) and a heating assembly (110) configured to heat the aerosol-generation substrate (200), comprising:
a heating cavity (147) comprising:
a first electrically conductive region;
a second electrically conductive region provided on a side of the first electrically conductive region; and
an insulating region provided between the first electrically conductive region and the second electrically conductive region and configured to electrically isolate the first electrically conductive region from the second electrically conductive region,
wherein the first electrically conductive region, the second electrically conductive region and the insulating region are configured so that when the aerosol-generation substrate (200) is accommodated in the heating cavity (147), an electrical connection is formed between the first electrically conductive region and the second electrically conductive region through the aerosol-generation substrate;
**characterised in that**
the electronic atomizer (100) further comprises a detection unit electrically connected to the heating assembly (110), and is configured to detect the capacitive sensing signal and the resistance signal between the first electrically conductive region and the second electrically conductive region, and to output an insertion signal according to the capacitive sensing signal and the resistance signal to cause the heating assembly (110) to generate heat to heat the aerosol-generation substrate (200).

2. The electronic atomizer (100) according to claim 1, wherein the heating cavity (147) comprises a first end and a second end that are arranged opposite to each other, the aerosol-generation substrate (200) is inserted into the second end from the first end, and the first electrically conductive region is provided on a side of the second electrically conductive region facing the second end.

3. The electronic atomizer (100) according to claim 2, wherein the first electrically conductive region is provided at the second end of the heating cavity (147), and the second electrically conductive region is provided on a cavity side wall of the heating cavity.

4. The electronic atomizer (100) according to claim 3, wherein one end of the second electrically conductive region is connected to the insulating region, and the other end of the second electrically conductive region extends to the first end of the heating cavity in a direction away from the insulating region.

5. The electronic atomizer (100) according to claim 1, further comprising a first structural member (141), a second structural member (143), and an insulating member (145), wherein the first structural member (141) forms a part of a cavity bottom wall of the heating cavity (147), and at least a part of the first structural member (141) forms the first electrically conductive region;
the second structural member (143) forms a part of a cavity side wall of the heating cavity (147), and at least a part of the second structural member (143) forms the second electrically conductive region; and
the insulating member (145) is arranged between the first structural member (141) and the second structural member (143), and the insulating member (145) forms the insulating region.

6. The electronic atomizer (100) according to claim 5, wherein the first structural member (141) is made of a conductive material, and/or the second structural member (143) is made of a conductive material.

7. The electronic atomizer (100) according to claim 5, wherein the insulating member (145) comprises an insulating bottom wall (1452) and an insulating side wall (1454) formed by extending from the insulating bottom wall (1452) in the same direction, the insulating side wall (1454) surrounds the insulating bottom wall (1452) in a circumferential direction to form an insulating accommodating cavity, the insulating bottom wall (1452) is provided with a mounting hole in communication with the insulating accommodating cavity, and the first structural member (141) is accommodated in the mounting hole.

8. The electronic atomizer (100) according to claim 7, wherein an inner diameter of the mounting hole is less than an inner diameter of the insulating accommodating cavity.

9. The electronic atomizer (100) according to any one of claims 1 to 8, comprising a battery assembly, wherein the heating assembly (110) is electrically connected to the battery assembly.

10. The electronic atomizer (100) according to claim 9, further comprising a vibration unit, wherein when the first structural member (141) and the second structural member (143) transmit electrical signals through the aerosol-generation substrate (200), the vibration unit vibrates.

## Patentansprüche

1. Elektronischer Zerstäuber (100), umfassend ein Aerosolerzeugungssubstrat (200) und eine Heizanordnung (110), die konfiguriert ist, um das Aerosolerzeugungssubstrat (200) zu erwärmen, umfassend:
einen Heizhohlraum (147), umfassend:
einen ersten elektrisch leitfähigen Bereich;
einen zweiten elektrisch leitfähigen Bereich, der auf einer Seite des ersten elektrisch leitfähigen Bereichs bereitgestellt ist; und
einen Isolierbereich, der zwischen dem ersten elektrisch leitfähigen Bereich und dem zweiten elektrisch leitfähigen Bereich vorgesehen und konfiguriert ist, um den ersten elektrisch leitfähigen Bereich von dem zweiten elektrisch leitfähigen Bereich elektrisch zu isolieren,
wobei der erste elektrisch leitfähige Bereich, der zweite elektrisch leitfähige Bereich und der Isolierbereich so konfiguriert sind, dass, wenn das Aerosolerzeugungssubstrat (200) in dem Heizhohlraum (147) aufgenommen ist, eine elektrische Verbindung zwischen dem ersten elektrisch leitfähigen Bereich und dem zweiten elektrisch leitfähigen Bereich durch das Aerosolerzeugungssubstrat gebildet ist;
**dadurch gekennzeichnet, dass**
der elektronische Zerstäuber (100) ferner eine Detektionseinheit umfasst, die elektrisch mit der Heizanordnung (110) verbunden und konfiguriert ist, um das kapazitive Erfassungssignal und das Widerstandssignal zwischen dem ersten elektrisch leitfähigen Bereich und dem zweiten elektrisch leitfähigen Bereich zu detektieren und ein Einführungssignal gemäß dem kapazitiven Erfassungssignal und dem Widerstandssignal auszugeben und somit zu bewirken, dass die Heizanordnung (110) Wärme erzeugt, um das Aerosolerzeugungssubstrat (200) zu erwärmen.

2. Elektronischer Zerstäuber (100) nach Anspruch 1, wobei der Heizhohlraum (147) ein erstes Ende und ein zweites Ende umfasst, die einander gegenüberliegend angeordnet sind, das Aerosolerzeugungssubstrat (200) von dem ersten Ende in das zweite Ende eingeführt ist und der erste elektrisch leitfähige Bereich auf einer Seite des zweiten elektrisch leitfähigen Bereichs, die dem zweiten Ende zugewandt ist, vorgesehen ist.

3. Elektronischer Zerstäuber (100) nach Anspruch 2, wobei der erste elektrisch leitfähige Bereich an dem zweiten Ende des Heizhohlraums (147) vorgesehen ist und der zweite elektrisch leitfähige Bereich an einer Hohlraumseitenwand des Heizhohlraums vorgesehen ist.

4. Elektronischer Zerstäuber (100) nach Anspruch 3, wobei ein Ende des zweiten elektrisch leitfähigen Bereichs mit dem Isolierbereich verbunden ist und sich das andere Ende des zweiten elektrisch leitfähigen Bereichs zu dem ersten Ende des Heizhohlraums in einer Richtung weg von dem Isolierbereich erstreckt.

5. Elektronischer Zerstäuber (100) nach Anspruch 1, ferner umfassend ein erstes Strukturelement (141), ein zweites Strukturelement (143) und ein Isolierelement (145), wobei das erste Strukturelement (141) einen Teil einer Hohlraumbodenwand des Heizhohlraums (147) bildet und mindestens ein Teil des ersten Strukturelements (141) den ersten elektrisch leitfähigen Bereich bildet;
das zweite Strukturelement (143) einen Teil einer Hohlraumseitenwand des Heizhohlraums (147) bildet und mindestens ein Teil des zweiten Strukturelements (143) den zweiten elektrisch leitfähigen Bereich bildet; und
das Isolierelement (145) zwischen dem ersten Strukturelement (141) und dem zweiten Strukturelement (143) angeordnet ist und das Isolierelement (145) den Isolierbereich bildet.

6. Elektronischer Zerstäuber (100) nach Anspruch 5, wobei das erste Strukturelement (141) aus einem leitfähigen Material hergestellt ist und/oder das zweite Strukturelement (143) aus einem leitfähigen Material hergestellt ist.

7. Elektronischer Zerstäuber (100) nach Anspruch 5, wobei das Isolierelement (145) eine isolierende Bodenwand (1452) und eine isolierende Seitenwand (1454) umfasst, die durch Verlängern von der isolierenden Bodenwand (1452) in der gleichen Richtung gebildet ist, die isolierende Seitenwand (1454) die isolierende Bodenwand (1452) in einer Umfangsrichtung umgibt, um einen isolierenden Aufnahmehohlraum zu bilden, die isolierende Bodenwand (1452) mit einem Montageloch in Verbindung mit dem isolierenden Aufnahmehohlraum vorgesehen ist und das erste Strukturelement (141) in dem Montageloch aufgenommen ist.

8. Elektronischer Zerstäuber (100) nach Anspruch 7, wobei ein Innendurchmesser des Montagelochs kleiner als ein Innendurchmesser des isolierenden Aufnahmehohlraums ist.

9. Elektronischer Zerstäuber (100) nach einem der Ansprüche 1 bis 8, umfassend eine Batterieanordnung, wobei die Heizanordnung (110) elektrisch mit der Batterieanordnung verbunden ist.

10. Elektronischer Zerstäuber (100) nach Anspruch 9, ferner umfassend eine Vibrationseinheit, wobei, wenn das erste Strukturelement (141) und das zweite Strukturelement (143) elektrische Signale durch das Aerosolerzeugungssubstrat (200) übertragen, die Vibrationseinheit vibriert.

## Revendications

1. Atomiseur électronique (100), comprenant un substrat de génération d'aérosols (200) et un assemblage de chauffe (110) configuré pour chauffer le substrat de génération d'aérosols (200), comprenant :
une cavité de chauffe (147) comprenant :
une première région électriquement conductrice ;
une seconde région électriquement conductrice prévue sur un côté de la première région électriquement conductrice ; et
une région d'isolation prévue entre la première région électriquement conductrice et la seconde région électriquement conductrice, et configurée pour isoler électriquement la première région électriquement conductrice de la seconde région électriquement conductrice,
dans lequel la première région électriquement conductrice, la seconde région électriquement conductrice et la région d'isolation sont configurées de telle sorte que, quand le substrat de génération d'aérosols (200) est logé dans la cavité de chauffe (147), une connexion électrique est formée entre la première région électriquement conductrice et la seconde région électriquement conductrice à travers le substrat de génération d'aérosols ;
**caractérisé en ce que**
l'atomiseur électronique (100) comprend en outre une unité de détection connectée électriquement à l'assemblage de chauffe (110), et est configuré pour détecter le signal de détection capacitif et le signal de résistance entre la première région électriquement conductrice et la seconde région électriquement conductrice, et pour sortir un signal d'insertion en accord avec le signal de détection capacitif et le signal de résistance pour amener l'assemblage de chauffe (110) à générer de la chaleur pour chauffer le substrat de génération d'aérosols (200).

2. Atomiseur électronique (100) selon la revendication 1, dans lequel la cavité de chauffe (147) comprend une première extrémité et une seconde extrémité qui sont agencées à l'opposé l'une de l'autre, le substrat de génération d'aérosols (200) est inséré dans la seconde extrémité depuis la première extrémité, et la première région électriquement conductrice est prévue sur un côté de la seconde région électriquement conductrice faisant face à la seconde extrémité.

3. Atomiseur électronique (100) selon la revendication 2, dans lequel la première région électriquement conductrice est prévue au niveau de la seconde extrémité de la cavité de chauffe (147), et la seconde région électriquement conductrice est prévue sur une paroi latérale de cavité de la cavité de chauffe.

4. Atomiseur électronique (100) selon la revendication 3, dans lequel une extrémité de la seconde région électriquement conductrice est connectée à la région d'isolation, et l'autre extrémité de la seconde région électriquement conductrice s'étend jusqu'à la première extrémité de la cavité de chauffe dans une direction en éloignement de la région d'isolation.

5. Atomiseur électronique (100) selon la revendication 1, comprenant en outre un premier élément structurel (141), un second élément structurel (143), et un élément d'isolation (145), dans lequel le premier élément structurel (141) forme une partie d'une paroi de fond de cavité de la cavité de chauffe (147), et au moins une partie du premier élément structurel (141) forme la première région électriquement conductrice ;
le second élément structurel (143) forme une partie d'une paroi latérale de cavité de chauffe (147), et au moins une partie du second élément structurel (143) forme la seconde région électriquement conductrice ; et
l'élément d'isolation (145) est agencé entre le premier élément structurel (141) et le second élément structurel (143), et l'élément d'isolation (145) forme la région d'isolation.

6. Atomiseur électronique (100) selon la revendication 5, dans lequel le premier élément structurel (141) est fait en matériau conducteur, et/le second élément structurel (143) est fait en matériau conducteur.

7. Atomiseur électronique (100) selon la revendication 5, dans lequel l'élément d'isolation (145) comprend une paroi de fond d'isolation (1452) et une paroi latérale d'isolation (1454) formée par extension depuis la paroi de fond d'isolation (145) dans la même direction, la paroi latérale d'isolation (1454) entoure la paroi de fond d'isolation (1452) dans une direction circonférentielle pour former une cavité de logement d'isolation, la paroi de fond d'isolation (1452) est dotée d'un trou de montage en communication avec la cavité de logement d'isolation, et le premier élément structurel (141) est logé dans le trou de montage.

8. Atomiseur électronique (100) selon la revendication 7, dans lequel un diamètre intérieur du trou de montage est inférieur à un diamètre intérieur de la cavité de logement d'isolation.

9. Atomiseur électronique (100) selon l'une quelconque des revendications 1 à 8, comprenant un assemblage de batterie, dans lequel l'assemblage de chauffe (110) est connecté électriquement à l'assemblage de batterie.

10. Atomiseur électronique (100) selon la revendication 9, comprenant en outre une unité de vibration, dans lequel, quand le premier élément structurel (141) et le second élément structurel (143) transmettent des signaux électriques à travers le substrat de génération d'aérosols (200), l'unité de vibration vibre.
